# EUROPÄISCHE PATENTSCHRIFT

(11) **EP 3 065 678 B1**
(45) Veröffentlichungstag und Bekanntmachung des Hinweises auf die Patenterteilung: **22.08.2018**
(21) Anmeldenummer: 14812122.1
(22) Anmeldetag: 05.11.2014
(51) Int. Cl.: A61F 6/08

(54) **THERAPEUTISCHES PESSAR**
THERAPEUTIC PESSARY
PESSAIRE THÉRAPEUTIQUE

(30) Priorität: 07.11.2013 DE 102013018608
(43) Veröffentlichungstag der Anmeldung: 14.09.2016
(73) Patentinhaber: Tunn, Ralf, 10435 Berlin (DE)
(72) Erfinder: Tunn, Ralf, 10435 Berlin (DE)
(74) Vertreter: Wehlan, Helmut
(86) Internationale Anmeldenummer: PCT/EP2014/002957
(87) Internationale Veröffentlichungsnummer: WO 2015/067361

(56) Entgegenhaltungen:
- EP-A1- 0 084 755
- WO-A2-2008/008794
- GB-A- 315 163
- US-A- 2 310 564
- US-A- 5 771 899

## Beschreibung

Die Erfindung betrifft die Gestaltung eines therapeutischen Pessars als vaginale bzw. uterale Rückbildungs-Fixationshilfe nach Senkungsbeschwerden von Patientinnen.

### Stand der Technik

Vaginale einführbare Pessare gibt es im Sinne von Stützpessaren für Patientinnen mit Senkungsbeschwerden, um die sich gesenkte Gebärmutter bzw. Scheide passiv zu stützen. Diese Pessare werden im Wesentlichen als Würfel-, Ring- bzw. Siebschalen-Pessare angeboten und haben somit eine andere Indikationsstellung zur Anwendung und einen nicht vergleichbaren Wirkungsansatz.

Derartige Stützpessare gelten aber nur als passive Stützpessare und können nicht gezielt anatomische Haltestrukturen entlasten und punktuell "Schienen", also Kontaktpunkte zur Regeneration, wieder herstellen und sind nicht als Unterstützung der Beckenbodenrekonvaleszenz indiziert.

Mittels der handelsüblichen Stützpessare kann die Hernie im Beckenboden abgedichtet werden, um somit zu verhindern, dass Harnblase, Enddarm bzw. Gebärmutter durch die Beckenbodenöffnung hindurch rutschen können. Sie üben aber lediglich eine passive Haltefunktion aus, dichten zwar weitgehend ab, aber unterstützen aktiv keine aktive Beckenbodenrekonvaleszenz. Wenn die Stützpessare entfernt werden, stellt sich der alte Zustand schnell wieder ein. Die Stützpessare haben keinen Einfluss auf gezielte anatomische Strukturen und besitzen keine Nachhaltigkeit.

Harninkontinenz und Deszensus sind gleichermaßen bekannte und gefürchtete postpartale Komplikationen. Die Häufigkeit der postpartalen Belastungsinkontinenz wird mit ca. 20 % angegeben. Realistische Zahlen für einen Descensus unteri sind kaum nachweisbar.

Die Ursachen sind multifaktoriell neurogen, myogen bzw. kollagenerg und können bis heute ätiologisch nicht sicher zugeordnet werden. Durch Bewußtseinsschulung der vielfältigen Ursachen und durch Konditionierung des Beckenbodens können myogene Defekte therapiert werden. Im Rahmen der präventiven und therapeutischen Rückbildungsgymnastik sind diese therapeutischen Strategien längst Standard und werden zunehmend flächendeckend angeboten. Neurogene Defekte können durch Elektrostimulation in ihrer Rekonvaleszenz unterstützt werden. Bei postpartaler Harninkontinenz bzw. Deszensus wird Elektrostimulation durch Physiotherapeuten und Frauenärzte mit unbefriedigten Erfolg, verordnet.

Defekte des bindegewebigen Halteapparates hingegen werden nur beim ausgeprägten Uterovaginalprolaps durch eine Pessartherapie im Sinne einer passiven Stützfunktion abgefangen. Bleiben derartige konservative Therapien bei Harninkontinenz und Deszensus ohne Erfolg und ist des weiteren der Leidensdruck hoch, werden operative Strategien diskutiert. Diese bestehen darin, überdehntes Bindegewebe zu raffen bzw. durch permanenten Gewebeersatz zu stabilisieren. Insbesondere bei Frauen im reproduktiven Alter sollte die Indikationsstellung zur operativen Rekonstruktion nur sehr zurückhaltend gestellt werden, da weitere Schwangerschaften negativen Einfluss auf das vorherige Operationsergebnis haben können.

Vielfach werden daher Schwangerschaften per Kaiserschnitt beendet, was mehr Sicherheit bezüglich des Erhalts der Bindegewebsfestigkeit zur ausreichenden Fixation von Uterus und Vagina gibt, aber andere Risiken mit sich bringen kann. Letztlich gibt es im Rahmen der postpartalen Rekonvalenszenz keine therapeutischen Konzepte zur Rekonvaleszenz des Erhaltes bzw. sicherer Rückgewinnung der notwendigen Festigkeit postpartaler Erschlaffung und Ermüdung des Bindegewebes zur ausreichenden Fixation von Uterus und Vagina in seiner naturgemäßen Halterungsstellung im kleinen weiblichen Becken.

Im Gegenteil, bei stillenden Müttern mit niedrigem Östrogenspiegel und die zusätzliche abdominale Druckbelastung auf Uterus und Vagina durch das Tragen eines Kindes haben letztlich negativen Einfluss auf die Rückbildung des Bindegewebes mit ausreichender Festigkeit zur Halterung von Uterus und Vagina.

Die WO 2008/008794 A2, EP 0 084 755 A1 und die US 5 771 899 A offenbaren ein Pessar gemäß dem Oberbegriff des Anspruchs 1.

### Aufgabe der Erfindung

Aufgabe der Erfindung ist es daher, ein therapeutisches Pessar oder dergleichen vaginale Einführhilfe zur Verfügung zu stellen, das bzw. die zur Therapie von Harninkontinenz und Deszensus, insbesondere nach Geburten und postpartalen Komplikationen verfügbar ist und auch prophylaktisch verwendbar ist, um vor dem Entstehen solcher Komplikationen weitgehend geschützt zu sein.

### Erfindungsgemäße Lösung der Aufgabe

Die Aufgabe wird erfindungsgemäß mit den Merkmalen des Anspruches 1 gelöst.

Vorteilhafte Ausführungsbeispiele ergeben sich aus den Merkmalen der Unteransprüche und der nachstehenden Beschreibung.

Das in die Vagina einer Patientin eingeführte erfindungsgemäße Pessar unterstützt in seiner vorgegebenen Lage in der Vagina erst Dank seiner erfindungsgemäßen räumlichen Struktur mit jeweils seiner zu erfindenden Aufgabe besonders gerecht werdenden und den gegebnen Verhältnissen anpassbarer Gestalt jeweils aktiv die Rekonvaleszenz der Bindegewebsstrukturen im Bereich seitlich der Harnröhre, die maßgeblich die notwendige Kraftübertragung vom Beckenboden auf die Harnröhre realisieren und somit den aktiven Kontinenzmechanismus, zum Halten des Urins unter körperlicher Belastung, unterstützen. Dieser Bereich wird durch das erfindungsgemäße Pessar nicht nur stabilisiert, sondern es entsteht ein gewisser Gegendruck, der das "Verkleben" der seitlichen Vaginalwand mit der Beckenbodenmuskulatur aktiv unterstützen soll. In ca. 10 % aller Nullipara, Frauen, die noch keine Schwangerschaften ausgetragen haben, ist diese Verklebung im Rahmen der MRT-Untersuchung anatomisch nur eingeschränkt darstellbar. Es besteht also ein konstitutionelles Risiko, insbesondere nach Schwangerschaft und Geburt, dass sich eine Harninkontinenz ausbilden kann. Diese Risikopatientinnen werden bis heute nicht differenziert und würden insbesondere durch die Nutzung des erfindungsgemäßen Pessars profitieren.

Ein weiteres nachhaltiges therapeutisches Ziel des erfindungsgemäßen Pessars eingeführt in das hintere Scheidengewölbe ist es, den Uterus in Höhe der Ligamenta sacrouterinae - Haltebänder, die von der Gebärmutter zum Kreuzbein verlaufen, zu reponieren und zu stabilisieren, um dadurch die wichtigsten Haltestrukturen der Gebärmutter in ihrer Rekonvaleszenz im Bereich des Beckenbodens aktiv zu unterstützen, mit einer Wirkung, die zur Ruhestellung mit einem Gipsverband vergleichbar ist. Dabei soll neben der Haltefunktion eine Kompression ausgeübt werden, die die Regenerationsprozesse der Bänder unterstützen soll, und zwar als ein aktives Therapiekonzept. Gipsverbände werden ca. 4 bis 6 Wochen angelegt, das Pessar soll 6 bis 12 Monate am Tage benutzt werden. Streng genommen kreuzt das Pessar den Beckenboden, hält vorne distal des Beckenboden die seitliche Befestigung der Vagina am Beckenboden fest. Hin zur Gebärmutter reponiert das Pessar die Gebärmutter oberhalb der Beckenbodenebene.

Die morphofunktionellen Schwachpunkte des Beckenbindegewebes im Rahmen von Schwangerschaft und Entbindung sind die myofasziale Verbindung der seitlichen Vaginalwand zum Muskel levator ani und die Ligg. sacrouterina. Die laterale Fixation der Vagina hat die Funktion der Kraftübertragung von der Levatormuskulatur zur Urethra im Sinne der aktiven Kontinenzerhaltung, die Ligg. sacrouterina stellen die wichtigste Komponente des uterinen Halteapparates dar. Kommt es zur Überdehnung und inkompletten Rückbildung dieser bindegewebigen Strukturen, sind Belastungsharninkontinenz und ein Descensus uteri die morphofunktionellen Konsequenzen.

Die Rückbildungs-Fixationshilfe wird in die Vagina eingeführt und soll entsprechend seiner Form beidseits im Bereich der lateralen Fixation der Vagina und der Ligg. sacrouterina (im Bereich des hinteren Scheidengewölbes) ein Widerlager bieten und somit aktiv für Druckentlastung sorgen. Das Widerlager richtet sich gegen den abdominalen Druck. Dadurch kommt es zur Kompression der lateralen Fixation der Vagina an die Levatormuskulatur, wodurch ein intrapartal verursachter Abriss dieser myofaszialen Strukturen vermieden wird und sich dadurch rekonnektieren bzw. regenerieren kann. Das gleiche gilt für die Ligg. sacrouterina. Diese werden durch die pessarbedingte Reponation des Uterus entlastet und können dadurch besser regenerieren.

Durch die Verkürzung der Ligg. sacrouterina kippt der Uterus aus seiner Streckstellung zurück in die Anteflexionshaltung. Dadurch wird der Uterus passiv durch Harnblase und Symphyse gehalten, was einen Deszensus-protektiven Effekt hat.

Bei der erfindungsgemäßen Rückbildungs-Fixationshilfe ist zum Einführen des erfindungsgemäßen Pessars in die Vagina (vergleichbar mit dem Einführen eines Tampons). Wiederverwendbarkeit ist gegeben. Das Material für das therapeutische Pessar besteht aus medizinischem Silikon. Die Patientin wechselt das Pessar bzw. die Fixationshilfe selbständig. Standard ist, es morgens das Pessar einzuführen und abends wieder zu entfernen, mit Leitungswasser zu reinigen und am folgenden Tag wieder einzuführen. Es wird vorzugsweise in drei Standardgrößen angeboten und kann entsprechend angepasst werden.

### Beschreibung einer Gestaltung eines erfindungsgemäßen therapeutischen Pessars

Die nachfolgende Beschreibung der beispielsweisen Gestaltung eines erfindungsgemäßen therapeutischen Pessars stellt für den Gynäkologen keine Beschränkung der Erfindung dar, die anhand der beigefügten Zeichnungen beschrieben ist. Hierzu zeigt:
- Fig. 1: ein erfindungsgemäßes therapeutisches Pessar gemäß einer beispielsweisen Ausgestaltung
- Fig. 2: die Anwendung des therapeutischen Pessars nach Fig. 1 in einer grob schematischen Seitenansicht in der Scheide (Vagina) einer Patientin.

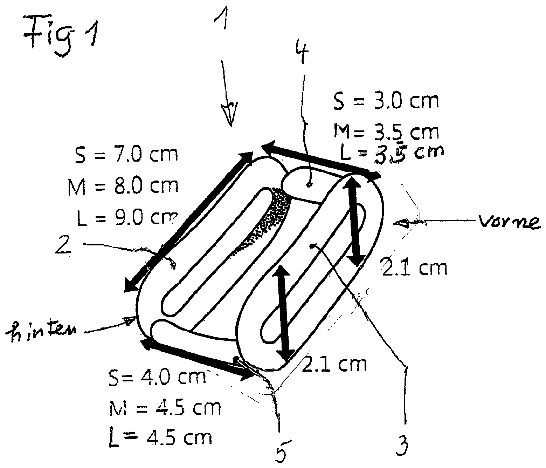

Ein erfindungsgemäßes therapeutisches Pessar nach Fig. 1 aus Kunststoff, vorzugsweise aus medizinischem Silikon, besteht aus wenigstens zwei schmalen, kufenartigen Teilen 3 und 4, die hier jeweils als längliche, ovale Ringkörper aus einem dünnen im Querschnitt runden schlauchförmigen Material aus Silikon gebildet sind, die von wenigstens zwei geraden Abstandshaltern am vorderen und hinteren Ende der Ringkörper auf Abstand gehalten sind. Dabei sind die Abstandshalter 4 und 5 jeweils mit ihren beiden Enden im mittleren Bereich der Rundungen der ovalen Ringkörper fest angeschlossen.

Der Abstand der ovalen Ringkörper am vorderen Ende ist etwas kleiner als der Abstand am hinteren Ende gewählt.

Der senkrechte Abstand der beiden parallel verlaufenden, länglichen Teile der ovalen Ringkörper ist jeweils gleich groß gehalten.

Bevorzugte Bemaßungen für die Länge der ovalen Ringkörper 2 und 3 betragen im Beispielsfall zwischen 7,0 bis 9,0 cm mit einer mittleren Länge von 8,0 cm. Die vertikalen Höhen der ovalen Ringkörper 2 und 3 betragen jeweils durchgehend 2,1 cm, wobei der Abstandshalter am vorderen Ende der ovalen Ringkörper zwischen 3 und 3,5 cm beträgt, ist der Abstand der Ringkörper am hinteren Ende mit 4,0 bis 4,5 cm bemessen.

Das Pessar besteht, wie dargestellt, aus vorzugsweise zwei schmalen, schlauchförmigen Abschnitten aus medizinischem Silikon, die einen gewählten/gleichbleibenden ringförmigen Querschnitt aufweisen, und eine gewählte Festigkeit und Elastizität besitzen.

Die vertikalen Höhen der Ringkörper 2 und 3 sind gleich und haben vorzugsweise jeweils eine Höhe von 2,1 cm.

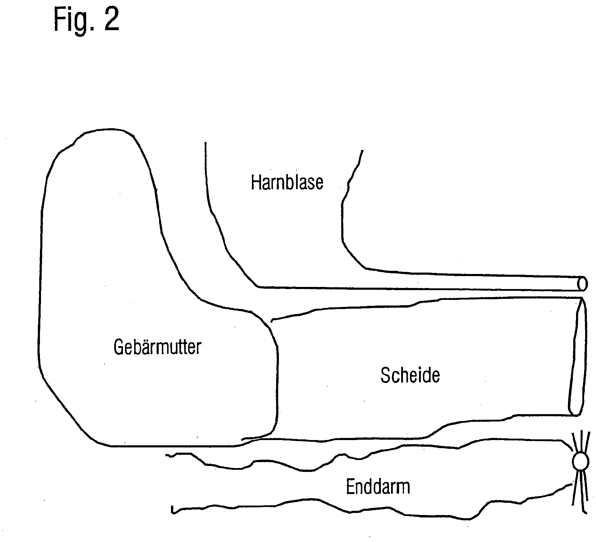

Fig. 2 zeigt in vereinfachter, schematischer Seitenansicht die Harnblase, die Gebärmutter, die Scheide und den Enddarm mit ihren Öffnungen an den unteren Enden und das erfinderische therapeutische Pessar in der Seitenansicht, das in die Scheide eingeführt ist.

Die beispielsweise Darstellung des erfinderischen, therapeutischen Pessars stellt keine Beschränkung der Erfindung dar.

Für den Gynäkologen bieten sich im Rahmen der Gestaltung des erfindungsgemäßen Pessars und der ihr zugrunde liegenden Zielsetzungen ohne Weiteres Varianten an, die im Bereich der Erfindung gleichwirkende Lösungen darstellen.

## Patentansprüche

1. Therapeutisches Pessar (1) oder vaginal eingeführte Hilfe zur aktiv wirksamen Therapie oder zur Prophylaxe bei drohender Harninkontinenz und/oder Descensus durch gezielte aktive Unterstützung der Rekonvaleszenz von geschwächten Beckenbindegewebsstrukturen, die für die vorstehenden Beschwerden im Wesentlichen verantwortlich sind, umfassend wenigstens zwei längliche kufenförmige Teile, die in einem wählbaren Abstand voneinander gehalten sind, **dadurch gekennzeichnet, dass** die kufenförmigen Pessarteile jeweils aus länglichen, ovalen Ringkörpern (2, 3) bestehen, die in gleicher Weise von in sich ringförmig geschlossen schlauchförmigen Abschnitten mit gewählten, kreisförmigen Durchmessern gebildet sind.

2. Therapeutisches Pessar (1) nach Anspruch 1, **dadurch gekennzeichnet, dass** die ovalen, ringförmigen Pessarteile jeweils an ihren einander gegenüber liegenden Enden von schlauchförmigen Abstandshaltern (4, 5) auf Abstand gehalten sind, die mit ihren Enden an die beiden kreisförmig gebogenen Endabschnitten der ovalen Ringkörper (2, 3) fest angeschlossen sind.

3. Therapeutisches Pessar (1) nach Anspruch 2, **dadurch gekennzeichnet, dass** der Abstand der kufenförmigen Pessarteile an ihren gegenüber liegenden, vorderen Enden um ein gewähltes Maß kürzer ist als der Abstand der kufenförmigen Pessarabschnitte an ihren gegenüber liegenden, hinteren Enden.

4. Therapeutisches Pessar (1) nach einem der vorstehenden Ansprüche 1 bis 3, **dadurch gekennzeichnet, dass** das Pessar aus medizinischem Silikon besteht, und dass das Pessar Abstandshalter besitzt mit denen die zwei länglichen kufenförmigen Teile auf Abstand von einander gehalten werden, wobei die längsseitigen kufenförmigen Pessarteile und die Abstandshalter aus schlauchförmigen Abschnitten mit gewählter Steifigkeit gebildet sind.

5. Therapeutisches Pessar (1) nach einem der vorstehenden Ansprüche 1 bis 4, **dadurch gekennzeichnet, dass** zwei kufenförmige Pessarteile vorgesehen sind, die jeweils die gleiche vertikale Höhe (vorzugsweise von 2,1 cm) und jeweils vorzugsweise eine Länge von 7,0 bis 9,0 cm aufweisen , und dass das Pessar Abstandshalter besitzt mit denen die zwei länglichen kufenförmigen Teile auf Abstand von einander gehalten werden, wobei der vordere kürzere Abstandshalter (4) zwischen den vorderen Pessarteilen vorzugsweise eine Länge von 3,0 bis 3,5 cm aufweist und der längere hintere Abstandshalter (5) an den hinteren Pessarteilen vorzugsweise eine Länge zwischen 4,0 bis 4,5 cm aufweist.

## Claims

1. A therapeutic pessary (1) or a vaginal insertion aid for the active therapy or prophylaxis during the risk of urinary incontinence and/or descensus by actively supporting the convalescence of weakened pelvic connective tissue structures, which are substantially responsible for the aforementioned symptoms, in a controlled manner, comprising at least two elongated, skid-shaped parts, which are held at a selectable distance from each other, **characterised in that** the skid-shaped pessary parts are each comprised of elongated, oval ring bodies (2, 3) which are formed in the same way by tubular sections annularly closed in themselves, with selected, circular diameters.

2. The therapeutic pessary (1) according to claim 1, **characterised in that** the oval, annular pessary parts are each kept at a distance by tubular spacers (4, 5) on their opposite ends which spacers with their two ends are firmly connected to the two circularly bent end sections of the oval ring bodies (2, 3).

3. Therapeutic pessary (1) according to claim 2, **characterised in that** the distance of the skid-shaped pessary parts on their opposite front ends is shorter by a selected dimension than the distance of the skid-shaped pessary sections on their opposite rear ends.

4. Therapeutic pessary (1) according to any one of the preceding claims 1 to 3, **characterised in that** the pessary is comprised of medical-grade silicone, and that the pessary comprises spacers by means of which the two elongated, skid-shaped parts are kept at a distance from each other, wherein the longitudinal, skid-shaped pessary parts and the spacers are formed from tubular sections with selected stiffness.

5. Therapeutic pessary (1) according to any one of the preceding claims 1 to 4, **characterised in that** two skid-shaped pessary parts are provided, which each comprise the same vertical height (preferably 2.1 cm) and each preferably a length of 7.0 to 9.0 cm, and that the pessary comprises spacers by means of which the two elongated, skid-shaped parts are kept at a distance from each other, wherein the front shorter spacer (4) between the front pessary parts preferably has a length of 3.0 to 3.5 cm, and the longer rear spacer (5) on the rear pessary parts preferably has a length between 4.0 and 4.5 cm.

## Revendications

1. Pessaire thérapeutique (1) ou un auxiliaire d'application vaginale destiné au traitement actif ou à la prévention des risques d'incontinence urinaire et/ou de prolapsus par un soutien actif ciblé de la récupération fonctionelle des structures tissulaires conjonctives affaiblies du bassin qui sont les principales responsables des troubles précités, comportant au moins deux éléments allongés en forme de patin qui sont tenus à une distance sélectionnable l'un de l'autre, **caractérisé en ce que** les éléments de pessaire en forme de patin chacun sont constitués des corps annulaires (2, 3) allongés ovales qui sont formés de la même manière des sections tubulaires fermées en soi en forme d'anneau avec des diamètres sélectionnés circulaires.

2. Pessaire thérapeutique (1) selon la revendication 1, **caractérisé en ce que** les éléments de pessaire ovales annulaires chacun sont tenus à une distance sur leurs extrémités opposées par des espaceurs tubulaires (4, 5) qui sont raccordés rigidement avec leurs extrémités aux deux parties terminales recourbées de manière circulaire des corps annulaires ovales (2, 3).

3. Pessaire thérapeutique (1) selon la revendication 2, **caractérisé en ce que** la distance des éléments de pessaire en forme de patin sur leurs extrémités opposées avant est plus court par une mesure sélectionnée que la distance des sections de pessaire en forme de patin sur leurs extrémités opposées arrière.

4. Pessaire thérapeutique (1) selon l'une quelconque des revendications précédentes 1 à 3, **caractérisé en ce que** le pessaire est constitué de silicone de qualité médicale et que le pessaire comporte des espaceurs avec lesquels les deux éléments allongés en forme de patin sont tenus à une distance l'un de l'autre, les éléments de pessaire longitudinaux en forme de patin et les espaceurs étant formés des sections tubulaires avec une rigidité sélectionnée.

5. Pessaire thérapeutique (1) selon l'une quelconque des revendications précédentes 1 à 4, **caractérisé en ce que** deux éléments de pessaire en forme de patin sont prévus ayant chacun la même hauteur verticale (de préférence 2,1 cm) et chacun de préférence une longueur de 7,0 à 9,0 cm, et que le pessaire comprend des espaceurs avec lesquels les deux éléments allongés en forme de patin sont tenus à une distance l'un de l'autre, l'espaceur (4) avant plus court entre les éléments de pessaire avant de préférence comportant une longueur de 3,0 à 3,5 cm et l'espaceur (5) arrière plus long sur les éléments de pessaire arrière de préférence comportant une longueur de 4,0 à 4,5 cm.
